# EUROPEAN PATENT APPLICATION

(11) **EP 4 574 976 A1**
(43) Date of publication of application: **25.06.2025**
(21) Application number: 23854816.8
(22) Date of filing: 04.08.2023
(51) Int. Cl.: C12N 15/11, A61K 31/712, A61K 48/00, C07H 21/00

(54) **NUCLEIC ACID FOR TRANSFECTION**

(30) Priority: 17.08.2022 JP 2022130213
(71) Applicant: AGC INC., Chiyoda-ku, Tokyo 1008405 (JP); The University of Tokyo, Bunkyo-ku, Tokyo 113-8654 (JP)
(72) Inventor: OKAZOE, Takashi, Tokyo 100-8405 (JP); OKAMOTO, Akimitsu, Tokyo 113-8654 (JP); MORIHIRO, Kunihiko, Tokyo 113-8654 (JP)
(74) Representative: Müller-Boré & Partner Patentanwälte PartG mbB
(86) International application number: PCT/JP2023/028633
(87) International publication number: WO 2024/038781

(57) **Abstract**

The present invention provides a nucleic acid for transfection having excellent cell membrane permeability, a method for producing the nucleic acid, and a transfection method. Specifically, the present invention provides a nucleic acid for transfection in which a nucleic acid targeted for introduction into a cell and a cell membrane-permeable group are linked together, the cell membrane-permeable group contains a perfluoroalkyl group having 2 to 10 carbons, and the perfluoroalkyl group may have one to five ether-bonded oxygen atoms between the carbon atoms, and also provides a nucleic acid transfection method that involves bringing the nucleic acid for transfection into contact with a cell to introduce the nucleic acid into the cell.

## Description

### [Technical Field]

The present invention relates to a nucleic acid for transfection having excellent cell membrane permeability, and a method for producing the same.

Priority is claimed on Japanese Patent Application No. 2022-130213, filed August 17, 2022, the content of which is incorporated herein by reference.

### [Background Art]

In recent years, research into nucleic acid drugs that use oligonucleotides continues to progress. Nucleic acid drugs have the excellent properties of exhibiting high specificity for the target molecule and having few side effects. However, nucleic acid drugs have poor cell membrane permeability, and delivering the drug to a target molecule located inside a cell is difficult. In particular, siRNA is double-stranded, and therefore has a higher molecular weight and a larger negative charge than antisense RNA, and inferior cell membrane permeability to antisense RNA, meaning a carrier is required for drug delivery. Known drug delivery agents include those that use lipid nanoparticles (Patent Document 1) and those that use cationic polymer nanoparticles (Patent Document 2). However, there remain many areas for improvement in terms of the efficiency of cell membrane permeability and toxicity concerns.

On the other hand, compounds having a polyfluoro structure are known to be stable and exhibit little toxicity in vivo, while offering excellent cellular uptake and endosomal escape (Non-Patent Document 1). Investigations are being conducted into utilizing these characteristics to introduce a polyfluoro structure as a portion having favorable cell membrane permeability into oligonucleotides and peptide nucleic acids (Patent Documents 3 and 4, Non-Patent Documents 2 to 5).

### [Citation List]

### [Patent Documents]

[Patent Document 1]
   International Patent Publication No. 2011/036557
[Patent Document 2]
   International Patent Publication No. 2017/212006
[Patent Document 3]
   International Patent Publication No. 2012/130941
[Patent Document 4]
   International Patent Publication No. 2021/060506
[Patent Document 5]
   Japanese Unexamined Patent Application, First Publication No. 2006-321797
[Patent Document 6]
   International Patent Publication No. 2000/056694

### [Non Patent Documents]

[Non Patent Document 1]
   Zhang et al., MRS Communications, 2018, vol. 8, pp. 303 to 313.
[Non Patent Document 2]
   Godeau et al., Medicinal Chemistry Communications, 2010, vol. 1. pp.76 to 78
[Non Patent Document 3]
   Ellipilli et al., Chemical Communications, 2016, vol. 52, pp. 521 to 524.
[Non Patent Document 4]
   Rochambeaua et al., Polymer Chemistry, 2016, vol. 7, pp. 4998 to 5003.
[Non Patent Document 5]
   Metelev et al., Theranostics, 2017, vol. 7, pp. 3354 to 3368.

### [Summary of Invention]

### [Technical Problem]

The nucleic acid with a bonded cell membrane-permeable group disclosed in Non-Patent Document 2 has a problem in that it cannot easily be synthesized by the phosphoramidite method.

Non-Patent Document 3 describes a peptide nucleic acid imparted with superior cell membrane permeability by incorporating a polyfluoro structure, but makes no mention of nucleic acids.

Non-Patent Document 4 discloses a nucleic acid with an added polyfluoro structure, but makes no mention of cell membrane permeability, and uses a separate transfection reagent for intracellular introduction.

Non-Patent Document 5 discloses a nucleic acid with an added polyfluoro structure, but further improvements in the cell membrane permeability would be desirable.

The present invention has an object of providing a nucleic acid for transfection that exhibits excellent cell membrane permeability, and a method for producing such a nucleic acid.

### [Solution to Problem]

The inventors of the present invention discovered that by introducing a perfluoroalkyl group into a nucleic acid targeted for cell introduction, the cell membrane permeability of the nucleic acid could be improved and the nucleic acid could be introduced into the cell even without using a transfection reagent, thus enabling them to complete the present invention.

In other words, the present invention includes the following aspects.
[1] A nucleic acid for transfection in which a nucleic acid targeted for introduction into a cell and a cell membrane-permeable group are linked together,
   the cell membrane-permeable group contains a perfluoroalkyl group having 1 to 10 carbon atoms, and
   the perfluoroalkyl group may have one to five ether-bonded oxygen atoms between the carbon atoms.
[2] The nucleic acid for transfection according to [1], wherein
   the cell membrane-permeable group has a structure represented by general formula (A1) shown below: 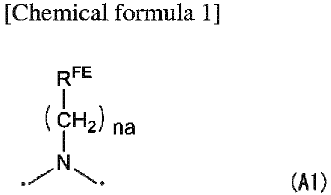 (wherein in the formula, R^{FE} represents a perfluoroalkyl group of 1 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, and the black dots represent bonding sites).
[3] The nucleic acid for transfection according to [1], wherein
   the cell membrane-permeable group has a structure represented by general formula (A2) shown below: 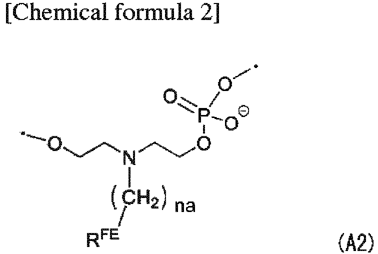 (wherein in the formula, R^{FE} represents a perfluoroalkyl group of 1 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, and the black dots represent bonding sites).
[4] The nucleic acid for transfection according to [3], having a structure in which two or more of the structures represented by the general formula (A2) are linked in tandem.
[5] A method for producing a nucleic acid for transfection, the method involving synthesizing the nucleic acid for transfection by bonding a compound represented by general formula (A2-0) shown below to a nucleic acid targeted for introduction into a cell using the phosphoramidite method: 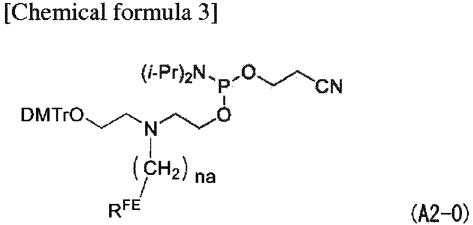 (wherein in the formula, R^{FE} represents a perfluoroalkyl group of 1 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, DMTr represents a 4,4'-dimethoxytriphenylmethyl group, and iPr represents an isopropyl group).
[6] The method for producing a nucleic acid for transfection according to [5], wherein two or more of the compounds represented by the general formula (A2-0) are bonded in tandem to the nucleic acid.
[7] A nucleic acid transfection method, the method involving bringing the nucleic acid for transfection according to any one of [1] to [4] into contact with a cell to introduce the nucleic acid into the cell.

### [Advantageous Effects of Invention]

The nucleic acid for transfection according to the present invention has an introduced perfluoroalkyl group, and therefore exhibits excellent cell membrane permeability. Accordingly, the nucleic acid for transfection can be introduced into a cell without requiring combination with another transfection reagent.

The production method according to the present invention enables production of the nucleic acid for transfection.

### [Brief Description of Drawings]

FIG. 1 is a diagram illustrating the results of measuring the mean fluorescent intensity at different culturing times of cells into which a fluorinated nucleic acid modified with N[C₈-PFC] at the 5'-terminal (PFC-ssDNA-FAM) and a control nucleic acid (ssDNA-FAM) have each been introduced in Example 1.
FIG. 2 illustrates fluorescent staining images of cells into which a fluorinated nucleic acid (PFC-ssDNA-FAM) has been introduced (left), and fluorescent staining images of cells into which a control nucleic acid (ssDNA-FAM) has been introduced using lipofectamine (right) in Example 1.

### [Description of Embodiments]

In the present invention and in this description, the term "nucleic acid" means a molecule in which nucleotides are bonded via phosphate diester bonds. These nucleotides include not only natural nucleotides (naturally occurring nucleotides) such as DNA and RNA, but also artificial nucleotides prepared by modifying a natural nucleotide to form a nucleotide capable of phosphate diester bonding with a natural nucleotide. Examples of artificial nucleotides include nucleotides in which a side chain or the like of a natural nucleotide has been modified with a functional group such as an amino group, nucleotides in which the hydroxyl group at the 2' position of a ribose skeleton has been substituted with a methoxy group, fluoro group, or methoxyethyl group or the like, phosphorothioate nucleotides (nucleotides in which the oxygen atom of a phosphate group has been substituted with a sulfur atom), morpholino nucleotides (nucleotides in which a ribose or deoxyribose has been substituted with a morpholine ring), BNA (Bridged Nucleic Acid), HNA (Hexitol Nucleic Acid), LNA (Locked Nucleic Acid), PNA (Peptide Nucleic Acid), TNA (Threose Nucleic Acid), GNA (Glycerol Nucleic Acid), and CeNA (Cyclohexenyl Nucleic Acid) and the like. Furthermore, the term "nucleic acid" also includes molecules in which only one or more natural nucleotides such as DNA or RNA are bonded via phosphate diester bonds, molecules in which one or more natural nucleotides and one or more artificial nucleotides are bonded via phosphate diester bonds, and only one or more artificial nucleotides are bonded via phosphate diester bonds.

In the present invention and in this description, "Cₚ₁₋ₚ₂" (wherein p1 and p2 are positive integers that satisfy p1<p2) means a group having p1 to p2 carbon atoms.

In the present invention and in this description, a "C₁₋₁₀ alkyl group" is an alkyl group having 1 to 10 carbon atoms, and may have either a linear chain or a branched chain. Similarly, a "C₂₋₁₀ alkyl group" is an alkyl group having 2 to 10 carbon atoms, and may have either a linear chain or a branched chain. Examples of the C₁₋₁₀ alkyl group include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, and decyl group.

In the present invention and in this description, a "C₁₋₆ alkyl group" is an alkyl group having 1 to 6 carbon atoms, and may have either a linear chain or a branched chain. Examples of the C₁₋₆ alkyl group include a methyl group, ethyl group, propyl group, isopropyl group, butyl group, isobutyl group, sec-butyl group, tert-butyl group, pentyl group, isopentyl group, neopentyl group, tert-pentyl group, and hexyl group.

In the present invention and in this description, an "alkylene group" is a divalent group in which two hydrogen atoms have been removed from a saturated hydrocarbon, and may have either a linear chain or a branched chain. Examples of the alkylene group include a methylene group, ethylene group, trimethylene group, tetramethylene group, pentamethylene group, hexamethylene group, heptamethylene group, octamethylene group, nonamethylene group, methylmethylene group, ethylmethylene group, methylethylene group, methylpropylene group, ethylethylene group, dimethylmethylene group, 1,2-dimethylethylene group, 1,1-dimethylethylene group, 1-ethylpropylene group, 2-ethylpropylene group, 1,2-dimethylpropylene group, 2,2-dimethylpropylene group, 1-propylpropylene group, 2-propylpropylene group, 1-methyl-1-ethylpropylene group, 1-methyl-2-ethylpropylene group, 1-ethyl-2-methylpropylene group, 2-methyl-2-ethylpropylene group, 1-methylbutylene group, 2-methylbutylene group, 3-methylbutylene group, 2-ethylbutylene group, 1-methylpentylene group, 2-ethylpentylene group, and 1-methylhexylene group.

In the present invention and in this description, a "C₁₋₁₀ perfluoroalkyl group" is a group in which all of the hydrogen atoms of an alkyl group having 1 to 10 carbon atoms have each been substituted with a fluorine atom. Examples of the C₁₋₁₀ perfluoroalkyl group include a perfluoromethyl group, perfluoroethyl group, perfluoropropyl group, perfluoroisopropyl group, perfluorobutyl group, perfluoroisobutyl group, perfluoro-sec-butyl group, perfluoro-tert-butyl group, perfluoropentyl group, perfluoroisopentyl group, perfluoroneopentyl group, perfluoro-tert-pentyl group, perfluorohexyl group, perfluoroheptyl group, perfluorooctyl group, perfluorononyl group, and perfluorodecyl group.

In the present invention and in this description, a "perfluoroalkylene group" is a group in which all of the hydrogen atoms of an alkylene group have each been substituted with a fluorine atom. Examples of the perfluoroalkylene group include groups in which all of the hydrogen atoms of the various alkylene groups listed above have each been substituted with a fluorine atom.

In the present invention and in this description, an "ether-bonded oxygen atom" describes an oxygen atom linking two carbon atoms, and does not include oxygen atoms that are linked in series to another oxygen atom. The number of ether-bonded oxygen atoms that an alkyl group of Nc carbon atoms (wherein Nc is an integer of 2 or greater) may have is a maximum of Nc-1. Further, a "C₂₋₁₀ alkyl group having an ether-bonded oxygen atom between carbon atoms" is a group having at least one ether-bonded oxygen atom between carbon atoms of the C₂₋₁₀ alkyl group. In the following description, the expression "ether bond-containing alkyl group" is also sometimes used.

In the present invention and in this description, a "C₂₋₁₀ perfluoroalkyl group having an ether-bonded oxygen atom between carbon atoms" is a group in which all of the hydrogen atoms of an ether bond-containing C₂₋₁₀ alkyl group (a group having at least one ether-bonded oxygen atom between carbon atoms of the C₂₋₁₀ alkyl group) have each been substituted with a fluorine atom. In the following description, this type of "perfluoroalkyl group having an ether-bonded oxygen atom between carbon atoms" is sometimes referred to as a "ether bond-containing perfluoroalkyl group".

Furthermore, in the following description, the expression "compound n" means a compound represented by formula (n).

The nucleic acid for transfection according to the present invention is a nucleic acid in which the nucleic acid targeted for introduction into a cell (the introduction target nucleic acid) and a cell membrane-permeable group are linked together. The introduction target nucleic acid typically has poor cell membrane permeability, but linking the cell membrane-permeable group improves the cell membrane permeability dramatically. Accordingly, the nucleic acid for transfection according to the present invention can be introduced into the cell interior simply by bringing the nucleic acid into contact with the cell surface, even without using a separate transfection reagent.

In the present invention and in this description, a "cell membrane-permeable group" means a group which, when added to a nucleic acid, improves the cell membrane permeability of that nucleic acid. Specific examples of these groups are groups containing a perfluoroalkyl groups having 1 to 10 carbon atoms (C1₋₁₀ perfluoroalkyl groups). Because perfluoroalkyl groups themselves exhibit excellent cell membrane permeability, by adding a group containing a perfluoroalkyl group to a nucleic acid, the cell membrane permeability of the nucleic acid can be improved.

The perfluoroalkyl group having cell membrane permeability may have one to five ether-bonded oxygen atoms between the carbon atoms. In other words, the perfluoroalkyl group having cell membrane permeability may be either a C₁₋₁₀ perfluoroalkyl group having no ether-bonded oxygen atoms between the carbon atoms, or a perfluoroalkyl group of 2 to 10 carbon atoms having one to five ether-bonded oxygen atoms between the carbon atoms (namely, an ether bonded oxygen-containing C₂₋₁₀ perfluoroalkyl group).

The C₁₋₁₀ perfluoroalkyl group having no ether-bonded oxygen atoms between the carbon atoms may have a linear chain or a branched chain. The cell membrane permeability of the perfluoroalkyl group improves as the number of carbon atoms increases, but if the number of carbon atoms is too great, then cytotoxicity may become a concern. A C₁₋₁₀ perfluoroalkyl group yields satisfactory cell membrane permeability, and can also suppress cytotoxicity. The cell membrane-permeable group of the nucleic acid for transfection according to the present invention is preferably a linear C₂₋₁₀ perfluoroalkyl group, more preferably a linear C₃₋₁₀ perfluoroalkyl group, and even more preferably a linear C₄₋₁₀ perfluoroalkyl group.

The ether bonded oxygen-containing C₂₋₁₀ perfluoroalkyl group is, for example, preferably a group represented by any of general formulas (F1) to (F3) shown below.
[Chemical formula 4]

-R^{F1}-O-R^{F2} (F1)

R^{F3}-(O-CF₂-CF₂-)n1-O-R^{F4} (F2)

-R^{F5}-(O-CF₂-CF₂-CF₂-)n2-O-R^{F6} (F3)

In general formula (F1), R^{F1} represents a perfluoroalkylene group having q1 carbon atoms and R^{F2} represents a perfluoroalkyl group having q2 carbon atoms. Further, q1 and q2 are natural numbers, the sum of which is at least 2 but not more than 10.

In general formula (F2), R^{F3} represents a perfluoroalkylene group having q3 carbon atoms and R^{F4} represents a perfluoroalkyl group having q4 carbon atoms. Further, n1, q3 and q4 are natural numbers for which the value of 2×n1+q3+q4 is at least 4 but not more than 10.

In general formula (F3), R^{F5} represents a perfluoroalkylene group having q5 carbon atoms and R^{F6} represents a perfluoroalkyl group having q6 carbon atoms. Further, n2, q5 and q6 are natural numbers for which the value of 3×n2+q5+q6 is at least 5 but not more than 10.

R^{F1} in general formula (F1), R^{F3} in general formula (F2) and R^{F5} in general formula (F3) may each represent a linear perfluoroalkylene group or a branched perfluoroalkylene group. Each of R^{F1} R^{F3} and R^{F5} is preferably a C₁₋₃ perfluoroalkylene group, more preferably a group in which all of the hydrogen atoms of a methylene group, ethylene group, methylmethylene group, ethylmethylene group, dimethylmethylene group or methylethylene group have each been substituted with a fluorine atom, even more preferably a group in which all of the hydrogen atoms of a methylene group, methylmethylene group, ethylmethylene group or dimethylmethylene group have each been substituted with a fluorine atom, and even more preferably a group in which all of the hydrogen atoms of a methylene group or a methylmethylene group have each been substituted with a fluorine atom.

R^{F2} in general formula (F1), R^{F4} in general formula (F2) and R^{F6} in general formula (F3) may each represent a linear perfluoroalkyl group or a branched perfluoroalkyl group. Each of R^{F2}, R^{F4} and R^{F6} is preferably a linear or branched C₁₋₅ perfluoroalkyl group, more preferably a linear C₁₋₅ perfluoroalkyl group, and even more preferably a linear C₂₋₄ perfluoroalkyl group.

The ether-bonded oxygen-containing C₂₋₁₀ perfluoroalkyl group in the cell membrane-permeable group of the nucleic acid for transfection according to the present invention is preferably a group of general formula (F1) in which R^{F1} is a group in which all of the hydrogen atoms of a methylene group or a methylmethylene group have each been substituted with a fluorine atom, and R^{F2} is a linear C₁₋₅ perfluoroalkyl group; a group of general formula (F2) in which R^{F3} is a group in which all of the hydrogen atoms of a methylene group or a methylmethylene group have each been substituted with a fluorine atom, n1 is 1, 2 or 3, and R^{F4} is a linear C₁₋₇ perfluoroalkyl group; or a group of general formula (F3) in which R^{F5} is a group in which all of the hydrogen atoms of a methylene group or a methylmethylene group have each been substituted with a fluorine atom, n2 is 1 or 2, and R^{F6} is a linear C₁₋₆ perfluoroalkyl group; and is more preferably a group of general formula (F1) in which R^{F1} is a group in which all of the hydrogen atoms of a methylene group or a methylmethylene group have each been substituted with a fluorine atom, and R^{F2} is a linear C₁₋₅ perfluoroalkyl group; a group of general formula (F2) in which R^{F3} is a group in which all of the hydrogen atoms of a methylene group or a methylmethylene group have each been substituted with a fluorine atom, n1 is 1, and R^{F4} is a linear C₁₋₆ perfluoroalkyl group; a group of general formula (F2) in which R^{F3} is a group in which all of the hydrogen atoms of a methylene group or a methylmethylene group have each been substituted with a fluorine atom, n1 is 2, and R^{F4} is a linear C₁₋₄ perfluoroalkyl group; a group of general formula (F2) in which R^{F3} is a group in which all of the hydrogen atoms of a methylene group or a methylmethylene group have each been substituted with a fluorine atom, n1 is 3, and R^{F4} is a linear C₁₋₃ perfluoroalkyl group; or a group of general formula (F3) in which R^{F5} is a group in which all of the hydrogen atoms of a methylene group or a methylmethylene group have each been substituted with a fluorine atom, n2 is 1, and R^{F6} is a linear C₁₋₆ perfluoroalkyl group.

In the present invention, the ether bond-containing perfluoroalkyl group introduced into the nucleic acid may, for example, use the ether bond-containing perfluoroalkyl group included in the fluorine compound disclosed in Patent Document 5 or 6 together with a production method therefor.

The cell membrane-permeable group of the nucleic acid for transfection according to the present invention preferably has a linking group for linking to the introduction target nucleic acid in addition to the perfluoroalkyl group that provides cell membrane permeability. There are no particular limitations on this linking group, and any group that enables linking of the nucleic acid and the perfluoroalkyl group may be used, including typical divalent or trivalent organic groups. Examples of the linking group include an alkylene group, alkenylene group, carbonyl group, amino group, ether linkage, thioether linkage, ester linkage, amide linkage, polyethylene glycol group (PEG: -(C₂H₄O)ₙ-), siloxane linkage, silyl ether linkage, a sugar, and a peptide. Further, groups in which two or three hydrogen atoms have been removed from a ring such as a pyrrole ring, pyrazole ring, imidazole ring, triazole ring, pyridine ring, pyrimidine ring, pyrazine ring, oxazole ring, thiazole ring, furan ring, thiophene ring or benzene ring may also be used as the linking group. Moreover, appropriate combinations of the above groups may also be used.

The cell membrane-permeable group of the nucleic acid for transfection according to the present invention preferably has, for example, a structure represented by general formula (A1) shown below. In general formula (A1), R^{FE} represents the ether bond-containing perfluoroalkyl group described above. Further, na represents an integer of 1 to 10. The black dots indicate bonding sites.

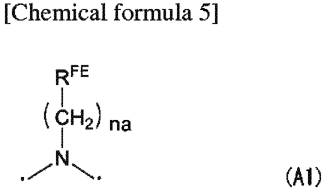

The cell membrane-permeable group of the nucleic acid for transfection according to the present invention is preferably a group that has been linked by any of various coupling reactions to the introduction target nucleic acid. For example, by performing the phosphoramidite method using a phosphoramidite containing the ether bond-containing perfluoroalkyl group as a raw material, the ether bond-containing perfluoroalkyl group can be easily introduced into the nucleic acid. Most widely used nucleic acid automatic synthesis devices utilize the phosphoramidite method. Accordingly, by using a phosphoramidite containing the ether bond-containing perfluoroalkyl group as a raw material, a nucleic acid having the ether bond-containing perfluoroalkyl group introduced at a desired location within a nucleic acid of any of various base sequences can be synthesized easily using an automatic synthesis device.

Examples of the phosphoramidite containing the ether bond-containing perfluoroalkyl group include compounds in which the nucleoside portion of any of the phosphoramidites typically used in nucleic acid synthesis has been substituted with an organic group containing the ether bond-containing perfluoroalkyl group. Examples of such compounds include compounds represented by general formula (A2-0) shown below.

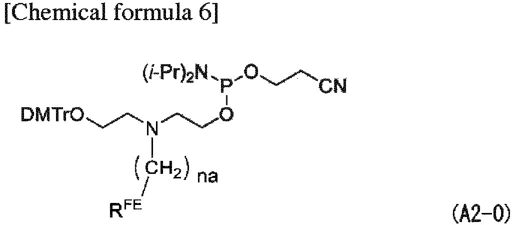

In general formula (A2-0), R^{FE} represents the ether bond-containing perfluoroalkyl group described above. Further, na represents an integer of 1 to 10, iPr represents an isopropyl group, and DMTr represents a 4,4'-dimethoxytriphenylmethyl group.

The nucleic acid synthesized by the phosphoramidite using the compound (A2-0) contains a structure represented by general formula (A2) shown below. In general formula (A2), R^{FE} represents the ether bond-containing perfluoroalkyl group described above. Further, na represents an integer of 1 to 10. The black dots indicate bonding sites.

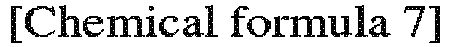

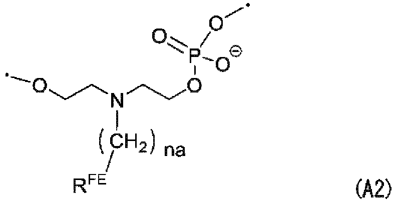

Nucleic acids in which two or more structures represented by the general formula (A2) are linked in tandem to the introduction target nucleic acid are also preferred as the nucleic acid for transfection according to the present invention. By incorporating a plurality of linked structures represented by general formula (A2), the cell membrane permeability of the introduction target nucleic acid can be improved dramatically. This type of nucleic acid for transfection can be synthesized by linking a plurality of the compound (A2-0) to the introduction target nucleic acid using the phosphoramidite method.

In addition, the cell membrane permeability can also be introduced into the introduction target nucleic acid using any of the methods disclosed in Non-Patent Documents 2 to 5.

The synthesized nucleic acid can be isolated and purified by any of various methods such as ion chromatography, gel permeation chromatography, reversed-phase chromatography, and normal phase chromatography.

There are no particular limitations on the introduction target nucleic acid within the nucleic acid for transfection according to the present invention, provided the nucleic acid requires introduction into a cell. A functional nucleic acid which exhibits some form of bioactivity when introduced into the target cell in vivo is preferred as the introduction target nucleic acid. Specific examples include nucleic acids for expressing a protein inside the cell, nucleic acids for suppressing gene expression inside the cell, nucleic acids for conducting genetic modification, nucleic acid aptamers that bind specifically to a target biomolecule, and functional nucleic acids that effect the physiological function of the cell. Examples of nucleic acids for protein expression include cDNA and mRNA that encode a protein, and the nucleic acid may be an expression plasmid vector incorporating a region that encodes the target protein. Examples of nucleic acids for suppressing gene expression include functional nucleic acids used in RNA interference such as siRNA, miRNA, shRNA, and antisense oligonucleotides, and RNAi vectors may also be used. Examples of nucleic acids for conducting genetic modification include genomic DNA fragments and the like. Examples of functional nucleic acids that effect the physiological function of the cell include decoy nucleic acids and CpG (cytosine-phosphate-guanine) oligonucleotides. In addition, nucleic acids having a functional molecule such as a fluorescent substance or a functional peptide or the like linked to the nucleic acid may also be used. For example, a nucleic acid molecule having a fluorescent substance linked to the terminal of a single-stranded nucleic acid that adopts a guanine quadruplex (G-quadruplex) may be used as the introduction target nucleic acid.

There are no particular limitations on the introduction target nucleic acid within the nucleic acid for transfection according to the present invention, and either a nucleic acid in which all of the included nucleotides are natural nucleotides or a nucleic acid in which some or all of the nucleotides are artificial nucleotides may be used. Further, the nucleic acid may be a single-stranded nucleic acid or a double-stranded nucleic acid. The nucleic acid may be a DNA, an RNA, or a chimeric nucleic acid of DNA and RNA.

In the nucleic acid for transfection according to the present invention, there are no particular limitations on the location at which the cell membrane-permeable group is introduced, and the group may be introduced at any location that does not impair the function of the nucleic acid. For example, the cell membrane-permeable group may be bonded to the 5'-terminal or the 3'-terminal of the introduction target nucleic acid, or may be introduced between two nucleotides within the introduction target nucleic acid.

The nucleic acid for transfection according to the present invention may be subjected to various modifications, provided that the cell membrane permeability provided by the perfluoroalkyl group is not impaired and the function of the introduction target nucleic acid is not impaired. Examples of these modifications include glycosylation, lipid modification, and peptide modification.

The nucleic acid for transfection according to the present invention contains a cell membrane-permeable group containing a perfluoroalkyl group, and therefore exhibits superior cell membrane permeability to a nucleic acid that does not contain such a group. Accordingly, the nucleic acid for transfection according to the present invention can be introduced into a cell simply by bring the nucleic acid into contact with the cell, without requiring the use of a typical transfection reagent such as lipofectamine. In those cases where the cell targeted for introduction of the nucleic acid for transfection according to the present invention is a cultured cell, the nucleic acid for transfection can be introduced into the cell simply by adding the nucleic acid for transfection to the culture medium and conducting cell culturing. Further, in the case of an animal tissue, the nucleic acid for transfection can be introduced into the cells constituting the tissue, for example, by spraying or coating a solution containing the dissolved nucleic acid for transfection according to the present invention onto the tissue surface.

### [Examples]

The present invention is described below using a series of examples, but the present invention is not limited to these examples.

### <Cell Cultures>

In the experiments described below, cell culturing was conducted in the following manner.

HeLa cells (from the RIKEN cell bank) were cultured in a low-glucose Dulbecco's Modified Eagle Medium (DMEM, manufactured by FUJIFIL Wako Pure Chemical Corporation) to which 10% of FBS and 0.5% of a mixed solution of penicillin-streptomycin had been added under a humidified atmosphere (5% by volume CO₂) at a temperature of 37°C.

Twenty four hours prior to the introduction of the nucleic acid into the cells, the cells were inoculated into a 96-well culture plate (manufactured by Bioramo LLC) at a concentration of 1.0 × 10⁴ cells/well, and subjected to adhesion and proliferation (preculturing).

Cells for image analysis were cultured on a 35 mm glass-bottom dish (manufactured by Iwaki Glass Co., Ltd.).

### [Synthesis Example 1]

A phosphoramidite containing a perfluoroalkyl group having 8 carbon atoms (C₈F₁₇-: hereinafter referred to as a C₈-PFC group) was synthesized, and using this phosphoramidite, a nucleic acid containing the C₈-PFC group was produced by the phosphoramidite method.

### <Synthesis of Phosphoramidite Containing C₈-PFC group>

The phosphoramidite containing the C₈-PFC group (N[Cs-PFC] amidite), represented by the formula shown below, was synthesized using the method disclosed in Non-Patent Document 4.

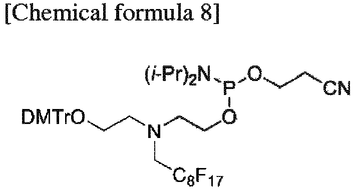

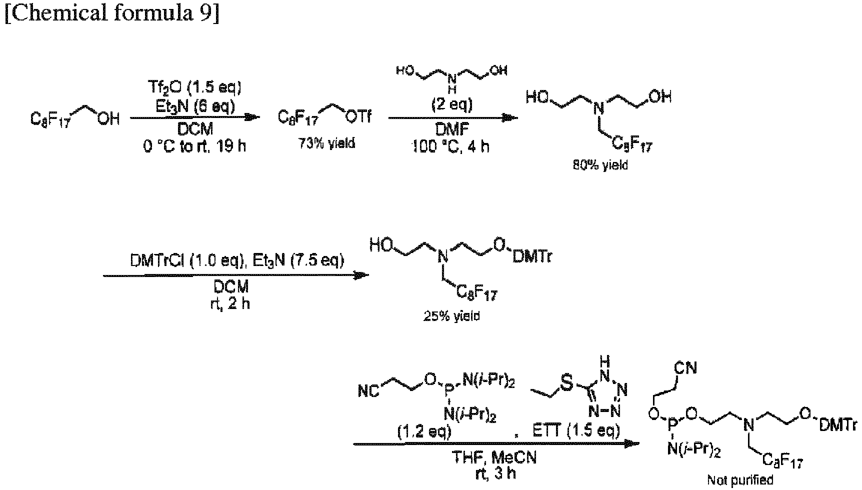

### [Synthesis Example 2]

A phosphoramidite containing a perfluoroalkyl group having 6 carbon atoms (CsF₁₇₋: hereinafter referred to as a C₆-PFC group) was synthesized, and using this phosphoramidite, a nucleic acid containing the C₆-PFC group was produced by the phosphoramidite method.

### <Synthesis of Phosphoramidite Containing C₆-PFC group>

The phosphoramidite containing the C₆-PFC group (N[C₆-PFC] amidite), represented by the formula shown below, was synthesized using the same method as Synthesis Example 1.

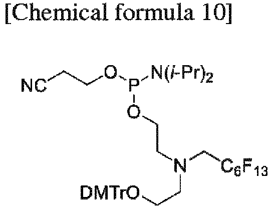

¹H-NMR (400 MHz, Chloroform-D) δ 7.45 to 7.39 (m, 2H), 7.35 to 7.26 (m, 6H), 7.23 to 7.17 (m, 1H), 6.85 to 6.79 (m, 4H), 3.83 to 3.52 (m, 6H), 3.79 (s, 6H), 3.79 (t, J = 16.6 Hz, 2H), 3.20 (t, J = 5.7 Hz, 2H), 2.94 (dt, J = 15.6, 5.9 Hz, 4H), 2.57 (t, J = 6.5 Hz, 2H), 1.16 (dd, J = 15.3, 6.8 Hz, 12H).

¹⁹F-NMR (376 MHz, Chloroform-D) δ -80.64 (t, J = 10.1 Hz, 3F), -117.31 to -117.72 (m, 2F), -121.86 to -121.91 (m, 2F), -122.55 to -122.94 (m, 2F), -123.32 to -123.40 (m, 2F), -125.93 to -126.06 (m, 2F).

³¹P-NMR (162 MHz, Chloroform-D) δ 148.35.

### [Synthesis Example 3]

A phosphoramidite containing a perfluoro polyether group having 5 carbon atoms (CF₃-(OCF₂)₄-: hereinafter referred to as a C₅-PFPE group) was synthesized, and using this phosphoramidite, a nucleic acid containing the C₅-PFPE group was produced by the phosphoramidite method.

### <Synthesis of Phosphoramidite Containing C₅-PFPE group>

The phosphoramidite containing the C₅-PFPE group (N[C₅-PFPE] amidite), represented by the formula shown below, was synthesized using the same method as Synthesis Example 1.

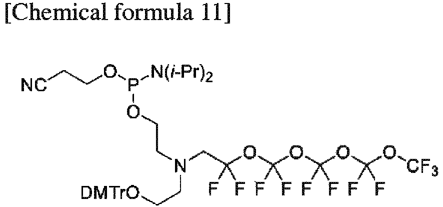

¹H-NMR (400 MHz, Chloroform-D) δ 7.44 to 7.39 (m, 2H), 7.35 to 7.27 (m, 6H), 7.23 to 7.17 (m, 1H), 6.89 to 6.75 (m, 4H), 3.82 to 3.45 (m, 6H), 3.79 (s, 6H), 3.27 (t, J = 11.1 Hz, 2H), 3.18 (t, J = 5.8 Hz, 2H), 2.93 (dt, J = 14.2, 6.0 Hz, 4H), 2.57 (tt, J = 6.5, 0.9 Hz, 2H), 1.15 (dd, J = 15.4, 6.8 Hz, 12H).

¹⁹F-NMR (376 MHz, Chloroform-D) δ -52.93 (p, J = 10.8 Hz, 2F), -54.85 (p, J = 10.1 Hz, 2F), -55.36 (qt, J = 9.4, 9.3 Hz, 2F), -56.76 (t, J = 8.8 Hz, 3F), -77.18 (td, J = 11.40, 11.39 Hz, 2F).

³¹P-NMR (162 MHz, Chloroform-D) δ 148.40.

### <DNA Synthesis>

DNA syntheses were conducted using commercially available reagents, various phosphoramidites (acetonitrile solutions, 0.1 M), and 5-ethylthio-1H-tetrazole (acetonitrile solution, 0.25 M) as an activator, using an NTS H-8 DNA/RNA synthesizer (manufactured by Nihon Techno Service Co., Ltd.).

### [Example 1]

The cellular uptake efficiency of a fluorescein-modified nucleic acid containing the C₈-PFC group and a fluorescein-modified nucleic acid that did not contain the C₈-PFC group was investigated by quantitative analysis using flow cytometry and by confocal microscopy.

### <Synthesis of Fluorescein-Modified Nucleic Acid>

A fluorescein-modified nucleic acid (ssDNA-FAM) having fluorescein introduced at the 3'-terminal of a single-stranded DNA (G-quadruplex DNA) comprising SEQ ID NO: 1 (5'-AGGGTTAGGGTTAGGGTTAGGG-3') was synthesized using the DNA synthesizer. Subsequently, one, two or five molecules of the N[C₈-PFC] amidite synthesized in Synthesis Example 1 were linked to the 5'-terminal of the obtained ssDNA-FAM by manual synthesis inside a glove box, thus obtaining an ssDNA-FAM having a cell membrane-permeable group containing two C₈-PFC groups linked in tandem to the 5'-terminal (namely, PFC-ssDNA-FAM).

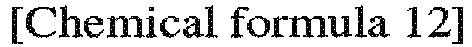

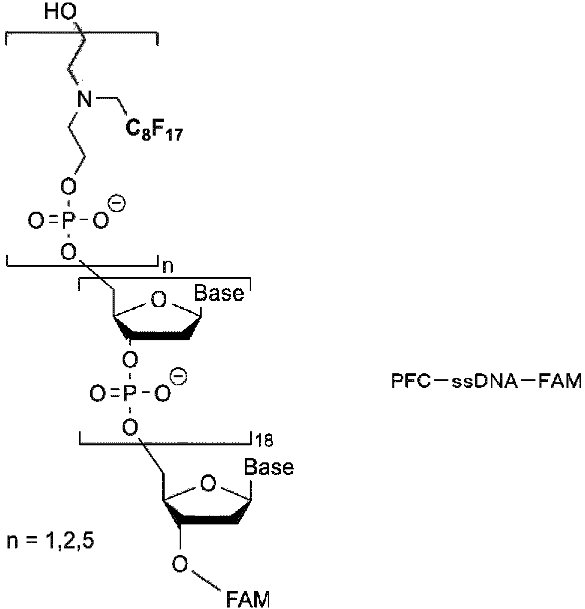

### <Preparation of Sample Solutions>

Solutions of ssDNA-FAM (control nucleic acid) and PFC-ssDNA-FAM (fluorinated nucleic acid) prepared in DMEM medium (low-glucose, 10% FBS, 1% mixed solution of penicillin-streptomycin) with concentrations of 1.0 µM were used as sample solutions. The 1.0 µM solution of ssDNA-FAM was used as a negative control, and a mixed solution containing the ssDNA-FAM and a commercially available transfection agent (Lipofectamine LTX, manufactured by Thermo Fisher Scientific Inc.) was used as a positive control. The mixing ratio and amounts used of the various reagents of the Lipofectamine LTX were in accordance with the standard protocol prescribed by the manufacturer. DMEM medium (low-glucose, 10% FBS, 1% mixed solution of penicillin-streptomycin) containing no introduced nucleic acid was used for blank measurements.

### <Evaluation of Cell Membrane Permeability by Flow Cytometry>

The medium of HeLa cells that had been cultured at 37°C for 24 hours was replaced with one of the sample solutions, and following culturing at 37°C for either one hour or four hours, the cell membrane permeability was evaluated. Following culturing for each of the prescribed time periods, the cell surfaces were washed three times with PBS (phosphate-buffered physiological saline solution), and the cells were then detached with trypsin-EDTA 0.05% (manufactured by Gibco Inc.) and collected. Subsequently, the collected cells were supplied to a flow cytometry system (Guava easyCyte (a registered trademark) 8), and the green-2 fluorescence (448 nm) used for detecting the fluorescent dye fluorescein (FAM) introduced into the synthesized nucleic acid was measured.

Cells prepared without adding the fluorescein-modified nucleic acid to the medium were used as a blank, cells prepared by adding only the control nucleic acid (ssDNA-FAM) to the medium were used as a negative control, and cells prepared by adding both the control nucleic acid and Lipofectamine LTX to the medium were used as a positive control.

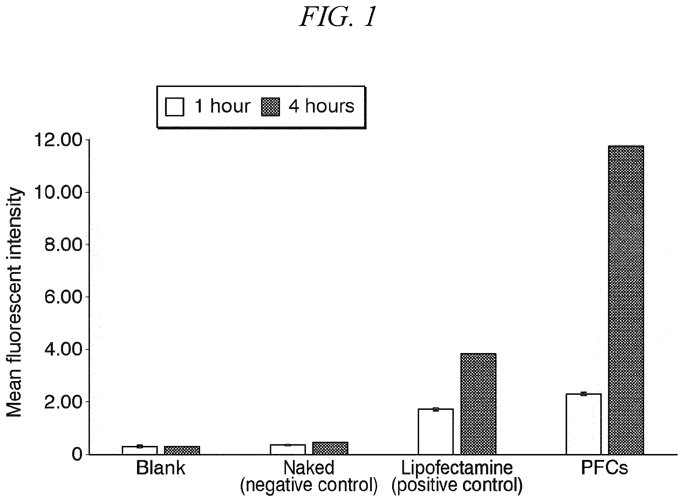

The mean fluorescent intensity of each cell type after each culturing time period is illustrated in FIG. 1. In the figure, the label "Blank" indicates the results for the cells prepared without adding a fluorescein-modified nucleic acid to the medium, the label "Naked" indicates the results for the cells prepared by adding only the control nucleic acid to the medium, the label "PFCs" indicates the results for the cells prepared by adding only the fluorinated nucleic acid (PFC-ssDNA-FAM comprising two molecules of N[C₈-PFC] amidite linked to the ssDNA-FAM) to the medium, and the label "Lipofectamine" indicates the results for the cells prepared by adding both the control nucleic acid and Lipofectamine LTX to the medium. As illustrated in FIG. 1, compared with the control nucleic acid having no added fluorine (ssDNA-FAM), the mean value for the fluorescent intensity of the fluorinated nucleic acid (PFC-ssDNA-FAM) was 6.7 times greater in the cells that had been cultured for one hour, and 24.4 times greater in the cells that had been cultured for four hours, and the mean fluorescent intensity for each group of cells was also higher than that observed for the samples that used lipofectamine. These results suggested that the nucleic acid having an introduced cell membrane-permeable group containing a perfluoroalkyl group had superior cell membrane permeability to the widely used transfection reagent.

Lipofectamine is currently a widely used transfection reagent, but requires the culture solution to be serum-free, and also requires the added medium to be replaced every few hours. Further, not only is it necessary to prepare a mixture of solutions for each experiment, but the variation in the permeability results for each experiment also tends to be large. In contrast, the nucleic acid for transfection according to the present invention containing a perfluoroalkyl group does not require a serum-free culture medium and does not require medium replacement, meaning it offers excellent properties as a reagent for introducing the nucleic acid into a cell.

### <Confocal Microscope Observations>

In order to confirm that the fluorinated nucleic acid had not merely adhered to the cell surface, but had rather been introduced into the interior of the cell, confocal microscope observations were conducted.

Sample solutions prepared in the same manner as described above were used. The medium of HeLa cells that had been cultured at 37°C for 24 hours was replaced with one of the sample solutions, and culturing was then conducted at 37°C for two hours. The cell surfaces were then washed three times with PBS, and nuclear staining was then performed by culturing the cells at 37°C for 30 minutes in Hoechst 33258 (manufactured by Dojindo Laboratories Co., Ltd.) that had been diluted 5,000-fold with OPTI-MEM medium. Subsequently, the cell surfaces were washed three times with PBS, the medium was replaced with Leibovitz's L-15 medium, and the cells were inspected using a confocal microscope (Spark SP8, manufactured by Leica AG).

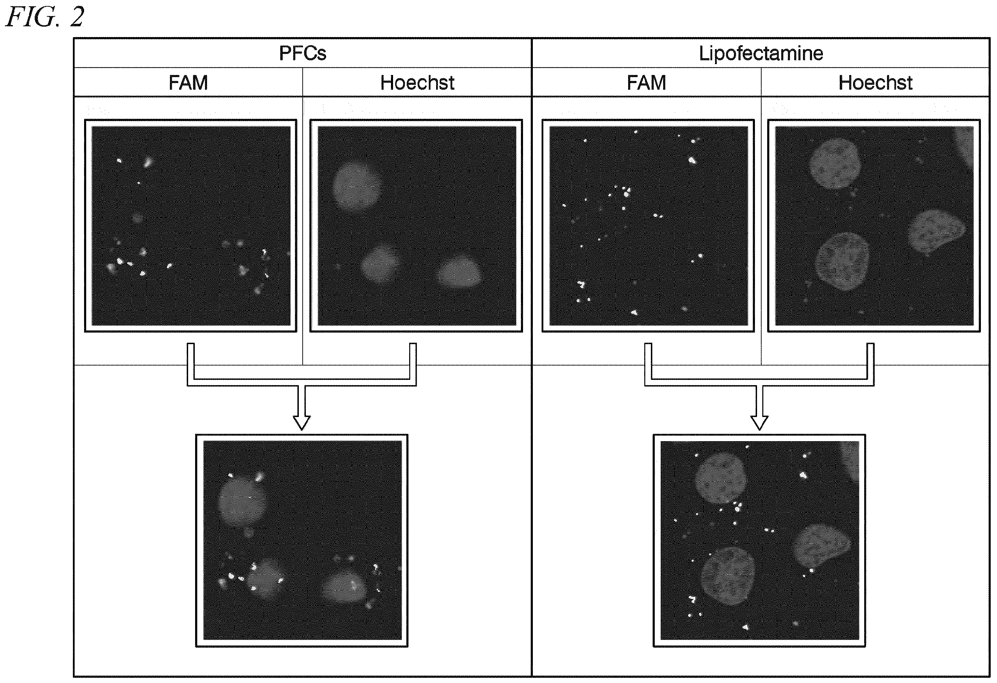

The fluorescent staining images of the cells into which the fluorinated nucleic acid (PFC-ssDNA-FAM) has been introduced are shown in FIG. 2 (left), and the fluorescent staining images of the cells into which the control nucleic acid (ssDNA-FAM) has been introduced using lipofectamine are shown in FIG. 2 (right). In the figure, the large circles in the Hoechst staining images are the nuclei, and the white dots in the FAM images represent the fluorescein labeling on the nucleic acid. In the case of the fluorinated nucleic acid, by two hours after starting the introduction process (medium replacement of the sample solution), the nucleic acid was confirmed as not being adhered to the cell surface, but rather clearly introduced into the interior of the cell. Moreover, in the case of the transfection using the mixed solution of lipofectamine and the control nucleic acid, the nucleic acid appeared as a circular shape wrapped in the lipid membrane inside the cell, and because those circular shapes were co-localized with the Hoechst nuclear staining, it is thought that much of the nucleic acid has formed an aggregate and become wrapped in the lipid membrane. (FIG. 2 (right)). On the other hand, in the case of the fluorinated nucleic acid, the nucleic acid exists as stand-alone points or strands, and is not co-localized with the Hoechst 33258 of the nuclear staining, suggesting that two hours after introduction, the nucleic acid has begun functioning rapidly inside the cytoplasm. Furthermore, a portion of the nucleic acid was confirmed as having entered the nuclei (FIG. 2 (left)). The above results suggest that the nucleic acid for transfection according to the present invention containing a perfluoroalkyl group can function as a functional nucleic acid for controlling siRNA or transfer, with this functionality occurring with quicker timing than nucleic acid introduction using lipofectamine reagent.

### [Example 2]

The cellular uptake efficiency of a fluorescein-modified nucleic acid containing a C₈-PFC group was investigated by quantitative analysis using flow cytometry.

### <Synthesis of Fluorescein-Modified Nucleic Acid>

A fluorescein-modified nucleic acid (ssDNA-FAM) having fluorescein introduced at the 3'-terminal of a single-stranded DNA comprising SEQ ID NO: 2 (5'-TTTTTCAGTTGACCATATA-3') was synthesized in a similar manner to Example 1. Subsequently, two molecules of the N[C₈-PFC] amidite synthesized in Synthesis Example 1 were linked to the 5'-terminal of the obtained ssDNA-FAM by manual synthesis inside a glove box, thus obtaining an ssDNA-FAM having a cell membrane-permeable group containing two C₈-PFC groups linked in tandem to the 5'-terminal (namely, PFC-ssDNA-FAM).

### <MTT Assay and LDH Assay>

The cell survival rate of the fluorinated nucleic acid (PFC-ssDNA-FAM) synthesized using the single-stranded DNA comprising SEQ ID NO: 2 was measured by MTT assay, and the cytotoxicity was measured by LDH assay. Cells prepared without adding the fluorescein-modified nucleic acid to the medium were used as a blank, cells prepared by adding only the control nucleic acid (ssDNA-FAM) to the medium were used as a negative control, and cells prepared by adding both the control nucleic acid and Lipofectamine LTX to the medium were used as a positive control.

HeLa cells were inoculated into a 96-well culture plate at a concentration of 1.0 × 10⁴ cells/well, subjected to adhesion and proliferation and then cultured for 24 hours, and the fluorescein-modified nucleic acid (fluorinated nucleic acid or control nucleic acid) was then added to the medium in sufficient amount to achieve a final concentration of 1 µM, and culturing was conducted for 24, 48 or 72 hours. Following culturing, the cell survival rate was measured by MTT assay, and the cytotoxicity was measured by LDH assay. The MTT assay was measured using a commercially available MTT cell count measurement kit (manufactured by Nacalai Tesque, Inc.) in accordance with the provided protocol. The LDH assay was measured using a commercially available cytotoxicity measurement kit (manufactured by Dojindo Laboratories Co., Ltd.) in accordance with the provided protocol.

The relative cell survival rate (expressed as a % relative to a value of 100% for the cells of the blank) representing the measurement results of the MTT assays are shown in Table 1, and the cytotoxicity rate (%) ([viable cell count of blank]) - [viable cell count of measurement target cells]) / [viable cell count of blank] × 100%) representing the measurement results of the LDH assays are shown in Table 2. In the tables, the label "Blank" indicates the results for the cells prepared without adding a fluorescein-modified nucleic acid to the medium, the label "Naked" indicates the results for the cells prepared by adding only the control nucleic acid to the medium, the label "PFCs" indicates the results for the cells prepared by adding only the fluorinated nucleic acid to the medium, and the label "Lipofectamine" indicates the results for the cells prepared by adding both the control nucleic acid and Lipofectamine LTX to the medium.

**[Table 1]**

| | Cell survival rate (%) | | | |
|---|---|---|---|---|
| Culture time | Blank | Naked | PFCs | Lipofectamine |
| 24 hours | 100 | 102.6 | 103.7 | 62.4 |
| 48 hours | 100 | 97.7 | 93.6 | 36.6 |
| 72 hours | 100 | 97.6 | 94.8 | 44.6 |

**[Table 2]**

| | Cytotoxicity rate (%) | | | |
|---|---|---|---|---|
| Culture time | Blank | Naked | PFCs | Lipofectamine |
| 24 hours | 0 | -0.6 | -1.9 | 35.9 |
| 48 hours | 0 | -2.7 | -3.0 | 35.1 |
| 72 hours | 0 | -2.8 | -5.1 | 54.8 |

As is evident from Tables 1 and 2, in the case of the medium to which both the control nucleic acid and the Lipofectamine LTX had been added, the cell survival rate had fallen to less than 50% after 72 hours of culturing. In contrast, the cells in the medium to which only the fluorescein-modified nucleic acid had been added exhibited a cell survival rate of greater than 90% for each culture time period. These results confirmed that unlike the case in which lipofectamine was used, the nucleic acid for transfection according to the present invention exhibited extremely low cytotoxicity.

### [Example 3]

The fluorinated nucleic acid (PFC-ssDNA-FAM) synthesized in Example 2 was confirmed as having high cell membrane permeability relative to HeLa cells, and therefore testing was conducted to confirm whether or not this superior permeability was also effective for other cell lines. The cell types used were HeLa cells, HepG2 cells (RIKEN cell bank), HEK293T cells (RIKEN cell bank), HT-1080 cells (JCRB cell bank), MCF7 cells (RIKEN cell bank), A549 cells (RIKEN cell bank), A431 cells (RIKEN cell bank), Huvec cells (Takara Bio Inc.), MDA-MB-231 cells (Angio-Proteomie), and MDA-MB-453 cells (RIKEN cell bank).

Cell culturing of the HeLa cells was conducted in the same manner as described for Example 1. The HepG2 cells, HEK293T cells and HT-1080 cells were cultured using the same medium and culturing conditions as the HeLa cells. The MCF7 cells were cultured using EMEM (Eagle's Minimum Essential Medium) (manufactured by FUJIFILM Wako Pure Chemical Corporation) to which 1% of non-essential amino acids (manufactured by Nacalai Tesque, Inc.), 1 mM of sodium pyruvate, 0.01 mg/mL of insulin (manufactured by FUJIFILM Wako Pure Chemical Corporation), 10% of FBS, and 1% of a mixed solution of penicillin-streptomycin had been added, under the same culturing conditions as the HeLa cells. The A549 cells were cultured using EMEM to which 1% of non-essential amino acids, 10% of FBS, and 1% of a mixed solution of penicillin-streptomycin had been added, under the same culturing conditions as the HeLa cells. The A431 cells were cultured using DMEM high-glucose (manufactured by FUJIFILM Wako Pure Chemical Corporation) to which 10% of FBS and 1% of a mixed solution of penicillin-streptomycin had been added as the medium, under the same culturing conditions as the HeLa cells. The Huvec cells were cultured in Endothelial Cell Growth Medium 2 (Supplement Pack Endothelial Cell GM2, manufactured by Takara Bio Inc.) to which 1% of a mixed solution of penicillin-streptomycin had been added, and with the exceptions of using 0.04% trypsin / 0.03% EDTA during cell detachment and using trypsin inhibitor / 0.1% BSA (manufactured by Takara Nio Inc.) for trypsin inactivation, the remaining culturing conditions were the same as those used for the HeLa cells. The MDA-MB-231 cells and the MDA-MB-453 cells were cultured in Leibovitz's L-15 medium, (manufactured by FUJIFILM Wako Pure Chemical Corporation) to which 10% of FBS and 1% of a mixed solution of penicillin-streptomycin had been added, in a carbon dioxide-free environment at 37°C.

Specifically, with the exception of adding the fluorinated nucleic acid (PFC-ssDNA-FAM) synthesized in Example 1 or the control nucleic acid (ssDNA-FAM) to the medium in sufficient amount to achieve a concentration of 1 µM, conducting culturing for 24 hours, and then supplying the cells to flow cytometry, the cell membrane permeability was investigated in the same manner as Example 1.

Based on the mean fluorescent intensity measured for each cell, a relative mean fluorescent intensity was determined relative to a value of 1 for the mean fluorescent intensity of the blank cells. This relative mean fluorescent intensity for each cell is shown in Table 3. Further, based on the results of the flow cytometry, the proportion (%) of the total number of cells into which the fluorescein-modified nucleic acid had been introduced (namely, the proportion of cells for which green fluorescence was confirmed) was determined, and is shown in Table 4. In the tables, the label "Blank" indicates the results for the cells prepared without adding a fluorescein-modified nucleic acid to the medium, the label "Naked" indicates the results for the cells prepared by adding only the control nucleic acid to the medium, the label "PFCs" indicates the results for the cells prepared by adding only the fluorinated nucleic acid to the medium, and the "Lipofectamine" column indicates the results for the cells prepared by adding both the control nucleic acid and Lipofectamine LTX to the medium.

**[Table 3]**

| | Relative mean fluorescent intensity | | | |
|---|---|---|---|---|
| | Blank | Naked | PFCs | Lipofectamine |
| MDA-MB-231 | 1 | 1.5 | 5.1 | 24.6 |
| MDA-MB-453 | 1 | 1.6 | 7.5 | 3.5 |
| Huvec | 1 | 3.1 | 146.6 | 7.4 |
| HepG2 | 1 | 1.2 | 4.3 | 2.7 |
| HeLa | 1 | 1.2 | 9.3 | 4.0 |
| A549 | 1 | 1.6 | 2.0 | 2.0 |
| A431 | 1 | 1.3 | 5.7 | 3.8 |
| HEK293T | 1 | 1.5 | 2.9 | 14.8 |
| HT-1080 | 1 | 1.5 | 5.5 | 7.2 |
| MCF7 | 1 | 1.9 | 3.4 | 7.6 |

**[Table 4]**

| | Proportion of cells having introduced nucleic acid (%) | | |
|---|---|---|---|
| | Naked | PFCs | Lipofectamine |
| MDA-MB-231 | 3 | 80 | 92 |
| MDA-MB-453 | 5 | 92 | 32 |
| Huvec | 28 | 100 | 64 |
| HepG2 | 1 | 35 | 23 |
| HeLa | 2 | 97 | 56 |
| A549 | 1 | 39 | 65 |
| A431 | 1 | 70 | 33 |
| HEK293T | 2 | 39 | 85 |
| HT-1080 | 2 | 72 | 74 |
| MCF7 | 25 | 82 | 74 |

As is evident from Table 3, regardless of the cell line, the mean fluorescent intensity of the cells prepared by adding the fluorinated nucleic acid (PFC-ssDNA-FAM) to the medium was higher than that of both the blank cells and the cells prepared by adding only the control nucleic acid to the medium, confirming that the fluorinated nucleic acid had been introduced into each of these cells. In particular, in a similar manner to that observed for the HeLa cells, the mean fluorescent intensity for the MDA-MB-453 cells, the Huvec cells, the HepG2 cells and the A431 cells was higher than that observed for the cells that used lipofectamine. Furthermore, as illustrated in Table 4, for many of the cells, the proportion of cells containing introduced nucleic acid among the cells prepared by adding the fluorinated nucleic acid (PFC-ssDNA-FAM) to the medium was similar to or higher than the proportion observed for the case using lipofectamine. These results confirmed that the nucleic acid for transfection according to the present invention exhibits superior cell membrane permeability to conventional lipofectamine reagents.

### [Example 4]

The cellular uptake efficiency of a fluorescein-modified nucleic acid containing the C₈-PFC group and a fluorescein-modified nucleic acid that did not contain the C₈-PFC group was investigated by quantitative analysis using flow cytometry.

### <Synthesis of Fluorescein-Modified Nucleic Acid>

A fluorescein-modified nucleic acid (ssDNA-FAM) having fluorescein introduced at the 3'-terminal of a single-stranded DNA comprising SEQ ID NO: 2 was synthesized in a similar manner to Example 1. Subsequently, one, two, three, four or five molecules of the N[C₈-PFC] amidite synthesized in Synthesis Example 1 were linked to the 5'-terminal of the obtained ssDNA-FAM in a similar manner to that described in Example 1, thus obtaining an ssDNA-FAM having a cell membrane-permeable group containing one, two, three, four or five C₈-PFC groups linked in tandem to the 5'-terminal (namely, PFC-ssDNA-FAM).

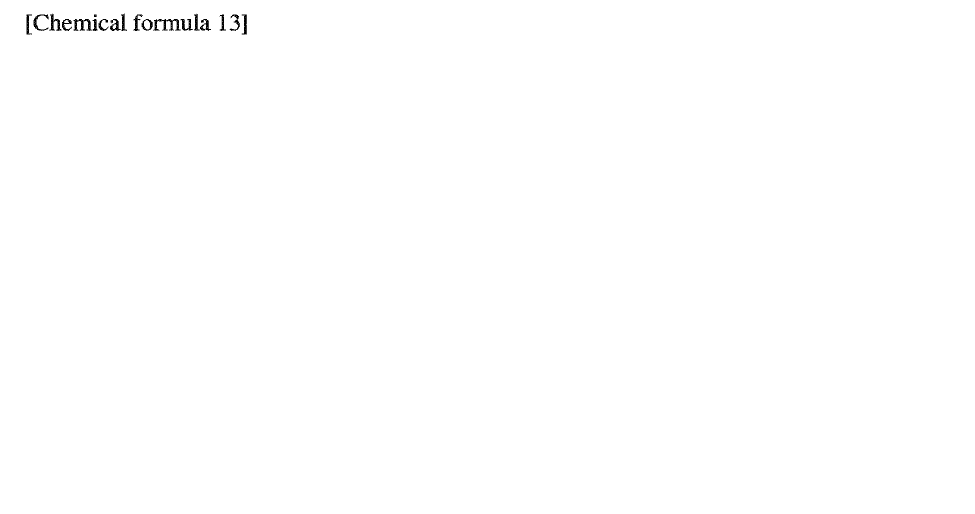

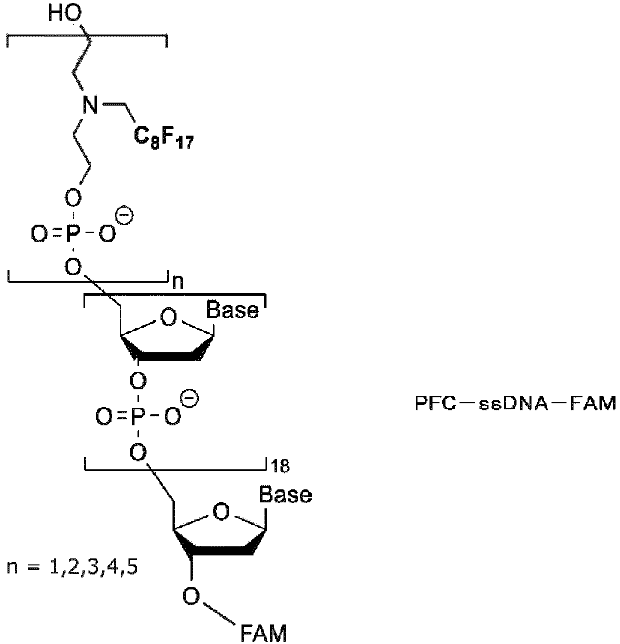

### <Preparation of Sample Solutions>

Solutions of ssDNA-FAM (the control nucleic acid) and PFC-ssDNA-FAM (the fluorinated nucleic acid) prepared in DMEM medium (low-glucose, 10% FBS, 1% mixed solution of penicillin-streptomycin) with concentrations of 1.0 µM were used as sample solutions. DMEM medium (low-glucose, 10% FBS, 1% mixed solution of penicillin-streptomycin) was also used for blank measurements.

### <Evaluation of Cell Membrane Permeability by Flow Cytometry>

In a similar manner to Example 1, HeLa cells were cultured in each of the sample solutions for 1 hour, 6 hours or 24 hours, and following culturing, the cell membrane permeability was evaluated. Cells prepared without adding the fluorescein-modified nucleic acid to the medium were used as a blank.

The mean fluorescent intensity (MFI) for each cell solution after each culturing time period is illustrated in Table 5. In the table, the label "Blank" indicates the results for the cells prepared without adding a fluorescein-modified nucleic acid to the medium, whereas the labels "N[PFC]n-DNA" indicate the results for the cells prepared by adding ssDNA-FAM containing n added molecules of fluorinated nucleic acid to the medium. As illustrated in Table 5, for the results observed after one hour, the mean value for the fluorescent intensity of the fluorinated nucleic acids (PFC-ssDNA-FAM) reached a maximum when n=2. These results suggest that in the case of the C₈-PFC group, the most superior cell membrane permeability is achieved when two-molecule modification is performed.

**[Table 5]**

| Mean fluorescent intensity (MFI) | 1h | | | 6h | | | 24h | | |
|---|---|---|---|---|---|---|---|---|---|
| | Data1 | Data2 | Data3 | Data1 | Data2 | Data3 | Data1 | Data2 | Data3 |
| Blank | 32.8 | 32.4 | 32.4 | 32.8 | 32.4 | 32.4 | 32.8 | 32.4 | 32.4 |
| N[PFC]ₒ-DNA | 35.8 | 36.3 | 36.4 | 32.5 | 31.6 | 36.4 | 31.8 | 31.9 | 32 |
| N[PFC]₁-DNA | 46.7 | 45.5 | 43.1 | 60 | 58.3 | 57.3 | 49 | 42.6 | 44.6 |
| N[PFC]₂-DNA | 364 | 372 | 409 | 462 | 481 | 513 | 60.6 | 59.9 | 60 |
| N[PFC]₃-DNA | 136 | 139 | 140 | 360 | 318 | 369 | 242 | 244 | 223 |
| N[PFC]₄-DNA | 130 | 131 | 138 | 474 | 462 | 458 | 329 | 342 | 423 |
| N[PFC]₅-DNA | 138 | 124 | 130 | 489 | 521 | 520 | 418 | 470 | 473 |

### [Example 5]

The cellular uptake efficiency of a fluorescein-modified nucleic acid containing the C₅-PFPE group was investigated by quantitative analysis using flow cytometry and by confocal microscopy.

### <Synthesis of Fluorescein-Modified Nucleic Acid>

A fluorescein-modified nucleic acid (ssDNA-FAM) having fluorescein introduced at the 3'-terminal of a single-stranded DNA comprising SEQ ID NO: 2 was synthesized in a similar manner to Example 1. Subsequently, two molecules of the N[C₅-PFPE] amidite synthesized in Synthesis Example 3 were linked to the 5'-terminal of the obtained ssDNA-FAM in a similar manner to that described in Example 1, thus obtaining an ssDNA-FAM having a cell membrane-permeable group containing two C₅-PFPE groups linked in tandem to the 5'-terminal (namely, PFPE-ssDNA-FAM).

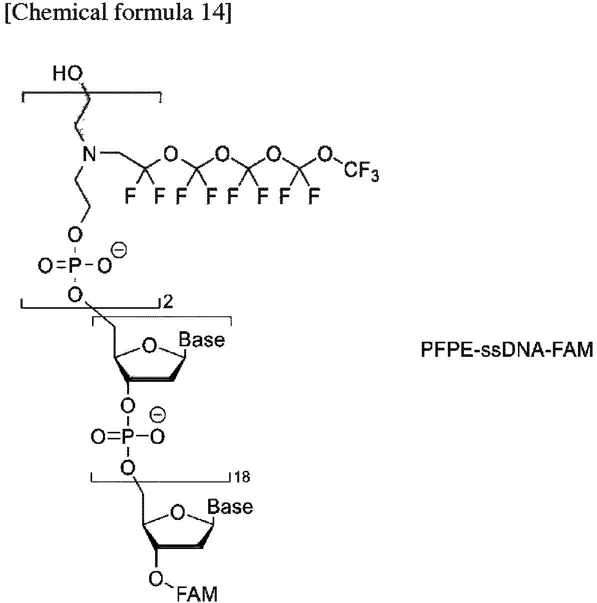

### <Preparation of Sample Solutions>

A solution of the PFPE-ssDNA-FAM (the fluorinated nucleic acid) prepared in DMEM medium (low-glucose, 10% FBS, 1% mixed solution of penicillin-streptomycin) with a concentration of 1.0 µM was used as a sample solution. DMEM medium (low-glucose, 10% FBS, 1% mixed solution of penicillin-streptomycin) was also used for blank measurements.

### <Evaluation of Cell Membrane Permeability by Flow Cytometry>

In a similar manner to Example 1, HeLa cells were cultured in each of the sample solutions at 37°C for 28 hours, and following culturing, the cell membrane permeability was evaluated. Cells prepared without adding the fluorescein-modified nucleic acid to the medium were used as a blank.

The mean fluorescent intensity (MFI) for each cell solution after the culturing period is illustrated in Table 6. In the table, the label "Blank" indicates the results for the cells prepared without adding the fluorescein-modified nucleic acid to the medium, and the label "C₅-PFPE" indicates the results for the cells prepared by adding ssDNA-FAM containing two added molecules of the fluorinated nucleic acid to the medium. As illustrated in Table 6, the mean value for the fluorescent intensity of the fluorinated nucleic acid (PFPE-ssDNA-FAM) was approximately twice that of the blank, indicating that superior cell membrane permeability could be achieved by modifying the nucleic acid with the C₅-PFPE group.

**[Table 6]**

| | Blank | C₅-PFPE |
|---|---|---|
| Mean fluorescent intensity (MFI) | 44 | 94 |

### [Industrial Applicability]

The present invention provides a nucleic acid for transfection that has cell membrane permeability attributable to a perfluoroalkyl group, and also provides a method for producing the nucleic acid for transfection. Because the nucleic acid for transfection according to the present invention exhibits excellent cell membrane permeability, and can therefore be introduced into a cell without requiring the use of a transfection reagent, it holds much promise for application in the fields of medicine and biotechnology.

## Claims

1. A nucleic acid for transfection in which a nucleic acid targeted for introduction into a cell and a cell membrane-permeable group are linked together,
the cell membrane-permeable group comprises a perfluoroalkyl group having 2 to 10 carbon atoms, and
the perfluoroalkyl group may have one to five ether-bonded oxygen atoms between the carbon atoms.

2. The nucleic acid for transfection according to Claim 1, wherein
the cell membrane-permeable group has a structure represented by general formula (A1) shown below: 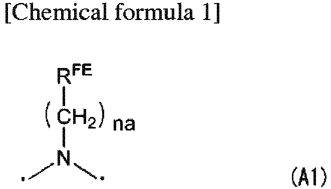 (wherein in the formula, R^{FE} represents a perfluoroalkyl group of 2 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, and black dots represent bonding sites).

3. The nucleic acid for transfection according to Claim 1, wherein
the cell membrane-permeable group has a structure represented by general formula (A2) shown below: 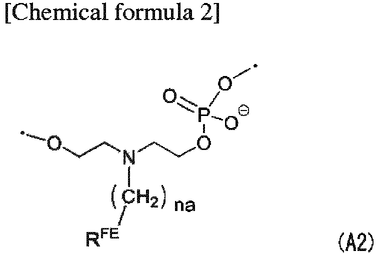 (wherein in the formula, R^{FE} represents a perfluoroalkyl group of 2 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, and black dots represent bonding sites).

4. The nucleic acid for transfection according to Claim 3, having a structure in which two or more structures represented by the general formula (A2) are linked in tandem.

5. A method for producing a nucleic acid for transfection, the method comprising synthesizing the nucleic acid for transfection by bonding a compound represented by general formula (A2-0) shown below to a nucleic acid targeted for introduction into a cell using a phosphoramidite method: 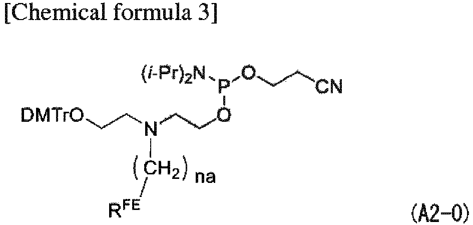 (wherein in the formula, R^{FE} represents a perfluoroalkyl group of 2 to 10 carbon atoms having no ether-bonded oxygen atoms between the carbon atoms, or a perfluoroalkyl group of 2 to 10 carbon atoms having one to five ether-bonded oxygen atoms between the carbon atoms, na represents an integer of 1 to 10, DMTr represents a 4,4'-dimethoxytriphenylmethyl group, and iPr represents an isopropyl group).

6. The method for producing a nucleic acid for transfection according to Claim 5, wherein two or more of the compounds represented by the general formula (A2-0) are bonded in tandem to the nucleic acid.

7. A nucleic acid transfection method, the method comprising bringing the nucleic acid for transfection according to any one of Claims 1 to 4 into contact with a cell to introduce the nucleic acid into the cell.
